(19) Europäisches Patentamt
European Patent Office
Office européen des brevets

(11) EP 0 802 192 A1

(12) # EUROPÄISCHE PATENTANMELDUNG

(43) Veröffentlichungstag: **22.10.1997 Patentblatt 1997/43**

(21) Anmeldenummer: **97105596.7**

(22) Anmeldetag: **04.04.1997**

(51) Int. Cl.$^6$: **C07D 233/58**, A61K 31/415, C07D 233/68, C07D 233/84, C07D 277/34, C07D 213/64, C07D 215/14, A61K 31/435, A61K 31/47

(84) Benannte Vertragsstaaten:
**AT BE CH DE DK ES FI FR GB GR IE IT LI LU MC NL PT SE**

(30) Priorität: **17.04.1996 DE 19615120**
**17.05.1996 DE 19619950**

(71) Anmelder: **BAYER AG**
**51368 Leverkusen (DE)**

(72) Erfinder:
- **Goldmann, Siegfried, Dr.**
  **42327 Wuppertal (DE)**
- **Müller, Ulrich, Dr.**
  **42111 Wuppertal (DE)**
- **Connell, Richard, Dr.**
  **06516 West Haven CT (US)**
- **Bischoff, Hilmar, Dr.**
  **42113 Wuppertal (DE)**
- **Denzer, Dirk, Dr.**
  **42115 Wuppertal (DE)**
- **Gruetzmann, Rudi, Dr.**
  **42657 Solingen (DE)**
- **Beuck, Martin, Dr.**
  **40699 Erkrath (DE)**

(54) **Heterocyclisch-substituierte Phenylglycinolamide mit antiatherosklerotischer Wirkung und Verfahren zu ihrer Herstellung**

(57) Die heterocyclisch-substituierte Phenylglycinolamide erhält man durch Umsetzung von heterocyclisch-substituierten Phenylessigsäuren mit entsprechenden Phenylglycinolen. Die heterocyclisch-substituierten Phenylglycinolamide eignen sich als Wirkstoffe in Arzneimitteln, insbesondere in antiatherosklerotische wirksamen Arzneimittel.

EP 0 802 192 A1

## Beschreibung

Die vorliegende Erfindung betrifft heterocyclisch-substituierte Phenylglycinolamide, Verfahren zu ihrer Herstellung und ihre Verwendung als Arzneimittel, insbesondere als antiatherosklerotische Arzneimittel.

Es ist bekannt, daß erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apoliprotein B-100 einhergeht. Es besteht daher nach wie vor ein starkes Bedürfnis, wirksame Arzneimittel zur Bekämpfung der Atherosklerose sowie koronarer Herzkrankheiten zur Verfügung zu stellen.

Die erfindungsgemäßen Verbindungen werden partiell vom breitesten Bedeutungsumfang der Publikationen DE 43 09 968, DE 43 02 956, DE 43 01 900, EP 565 086, EP 560 163, EP 560 162, EP 513 533 und DE 42 00 954 umfaßt, ohne daß dort ein pharmakologischer Vertreter dieser Art genannt wird. Die hier aufgeführten Verbindungen zeigen überraschenderweise eine Verminderung oder vollständige Inhibition der Bildung und/oder der Freisetzung von ApoB-100-assoziierter Lipoproteine aus Leberzellen.

Die vorliegende Erfindung betrifft heterocyclisch substituierte Phenylglycinolamide der allgemeinen Formel (I)

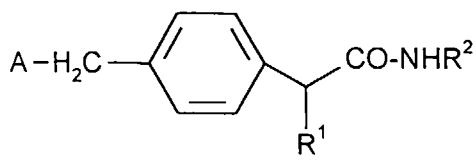

(I)

in welcher

A für Chinolyl oder für einen Rest der Formel

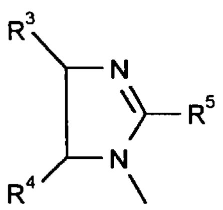 , 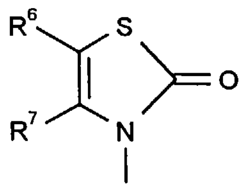 oder 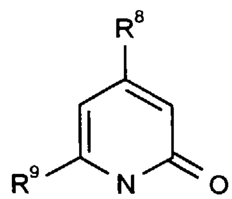

steht, in welcher

$R^3$, $R^4$, $R^6$ und $R^7$ gleich oder verschieden sind und Wasserstoff, Phenyl, Halogen, Formyl, Carboxyl, geradkettiges oder verzweigtes Alkoxycarbonyl mit bis zu 4 Kohlenstoffatomen oder geradkettiges oder verzweigtes Alkyl mit bis zu 4 Kohlenstoffatomen bedeuten, das gegebenenfalls durch Hydroxyl substituiert ist,

$R^5$ Phenyl, geradkettiges oder verzweigtes Alkyl, Acyl oder Alkylthio mit jeweils bis zu 6 Kohlenstoffatomen oder eine Gruppe der Formel $-CO-NR^{10}R^{11}$ bedeutet, worin

$R^{10}$ und $R^{11}$ gleich oder verschieden sind und Wasserstoff oder geradkettiges oder verzweigtes Alkyl mit bis zu 5 Kohlenstoffatomen bedeuten,

$R^8$ und $R^9$ gleich oder verschieden sind und Wasserstoff, geradkettiges oder verzweigtes Alkyl oder Alkoxycarbonyl mit jeweils bis zu 6 Kohlenstoffatomen oder einen Rest der Formel $-CO-R^{12}$ bedeuten, worin

$R^{12}$ Morpholinyl oder den Rest der Formel

$-NH-CH_2-C_6H_5$ oder

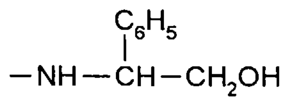

bedeutet,

$R^1$ für Cycloalkyl mit 3 bis 8 Kohlenstoffatomen steht, oder für geradketiges oder verzweigtes Alkyl mit bis zu 10 Kohlenstoffatomen steht,

$R^2$ für einen Rest der Formel

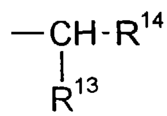

steht,
worin

$R^{13}$ Wasserstoff oder einen Rest der Formel $CH_2$-OH bedeutet,

$R^{14}$ Phenyl bedeutet, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

Die erfindungsgemäßen heterocyclisch-substituierten Phenylglycinolamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Bevorzugt sind erfindungsgemäße Verbindungen der allgemeinen Formel (I),
in welcher

A für Chinolyl oder für einen Rest der Formel

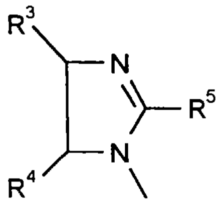
,
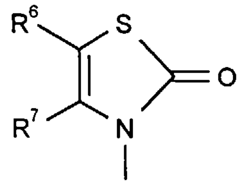
oder
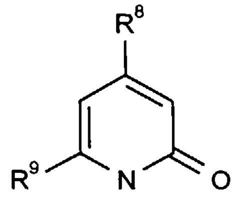

steht,
in welcher

| | |
|---|---|
| $R^3$, $R^4$, $R^6$ und $R^7$ | gleich oder verschieden sind und Wasserstoff, Phenyl, Fluor, Chlor, Brom, Formyl, geradkettiges oder verzweigtes Alkoxycarbonyl mit bis zu 3 Kohlenstoffatomen oder geradkettiges oder verzweigtes Alkyl mit bis zu 3 Kohlenstoffatomen bedeuten, das gegebenenfalls durch Hydroxyl substituiert ist, |
| $R^5$ | Phenyl, geradkettiges oder verzweigtes Alkyl, Acyl oder Alkylthio mit jeweils bis zu 5 Kohlenstoffatomen oder eine Gruppe der Formel $-CO-NR^{10}R^{11}$ bedeutet, worin |
| $R^{10}$ und $R^{11}$ | gleich oder verschieden sind und Wasserstoff oder geradkettiges oder verzweigtes Alkyl mit bis zu 5 Kohlenstoffatomen bedeuten, |
| $R^8$ und $R^9$ | gleich oder verschieden sind und Wasserstoff, geradkettiges oder verzweigtes Alkyl oder Alkoxycarbonyl mit jeweils bis zu 5 Kohlenstoffatomen oder einen Rest der Formel $-CO-R^{12}$ bedeuten, worin |
| $R^{12}$ | Morpholinyl oder den Rest der Formel |

$-NH-CH_2-C_6H_5$ oder

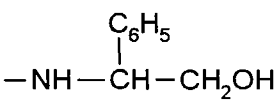

bedeutet,

| | |
|---|---|
| $R^1$ | für Cyclopropyl, Cyclobutyl, Cyclopentyl, Cyclohexyl oder Cycloheptyl steht, oder für geradkettiges oder verzweigtes Alkyl mit bis zu 8 Kohlenstoffatomen steht, |
| $R^3$ | für einen Rest der Formel |

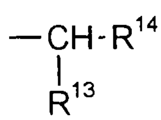

steht,
worin

| | |
|---|---|
| $R^{13}$ | Wasserstoff oder einen Rest der Formel $CH_2$-OH bedeutet, |
| $R^{14}$ | Phenyl bedeutet, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen substituiert ist, |

und deren Salze.

Besonders bevorzugt sind erfindungsgemäße Verbindungen der allgemeinen Formel (I), in welcher

A für Chinolyl oder für einen Rest der Formel

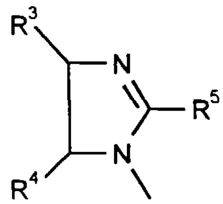 , 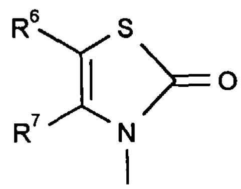 oder 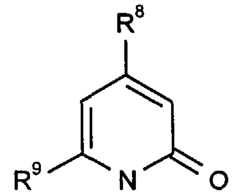

steht, in welcher

$R^3$, $R^4$, $R^6$ und $R^7$ gleich oder verschieden sind und Wasserstoff, Phenyl, Chlor, Formyl, Methoxycarbonyl, Ethoxycarbonyl oder Methyl oder Ethyl bedeuten, das gegebenenfalls durch Hydroxyl substituiert ist,

$R^5$ Phenyl, Methylthio, Acetyl, Ethylthio oder geradkettiges oder verzweigtes Alkyl mit bis zu 4 Kohlenstoffatomen oder eine Gruppe der Formel $-CO-NR^{10}R^{11}$ bedeutet, worin

$R^{10}$ und $R^{11}$ gleich oder verschieden sind und Wasserstoff oder geradkettiges oder verzweigtes Alkyl mit bis zu 3 Kohlenstoffatomen bedeuten,

$R^8$ Methyl, Methoxycarbonyl, Ethoxycarbonyl oder den Rest der Formel

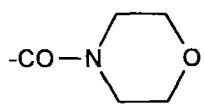 ,

$-CO—NH-CH_2—C_6H_5$ oder

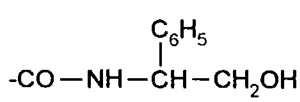

bedeutet, und

$R^9$ Wasserstoff, Methyl, Propyl oder Butyl bedeutet,

und deren Salze.

Ganz besonders bevorzugt sind Verbindungen der allgemeinen Formel (I), die in der nachfolgenden Tabelle aufgeführt sind.

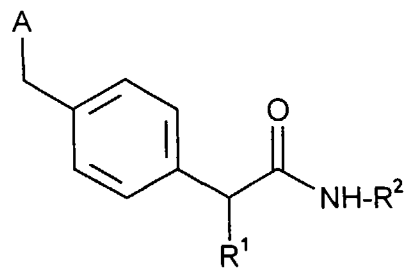

| A | $R^1$ | $R^2$ |
|---|---|---|
| 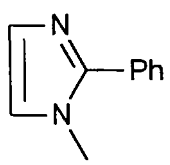 | (R&S) cPent | $CH_2$-$C_6H_5$ |
| 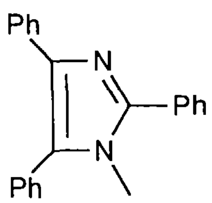 | (R&S) cPent | 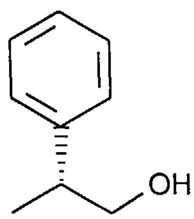 |
| 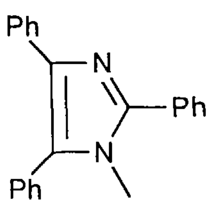 | (R&S) cPent | $CH_2$-$C_6H_5$ |
| 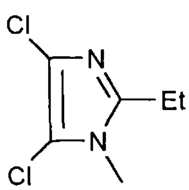 | (R&S) cPent | 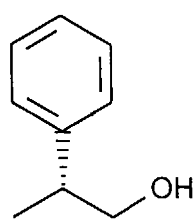 |
| 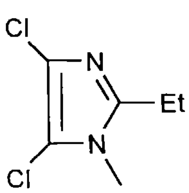 | (dia A) cPent | 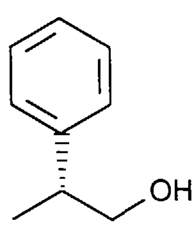 |

| A | $R^1$ | $R^2$ |
|---|---|---|
| 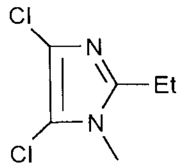 | (dia B) cPent | 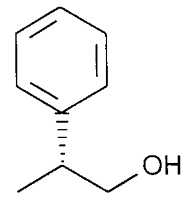 |
| 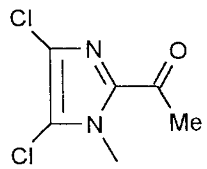 | (R&S) cPent | 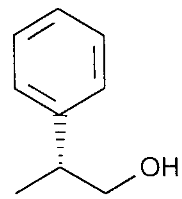 |
| 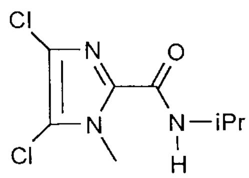 | (R&S) cPent | 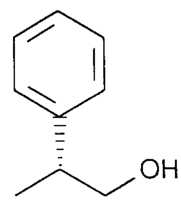 |
| 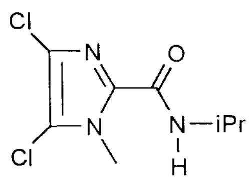 | (dia A) cPent | 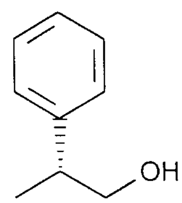 |
| 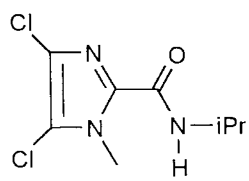 | (dia B) cPent | 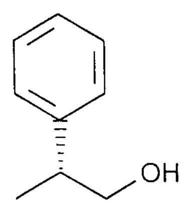 |
| 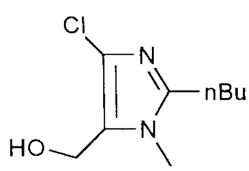 | (dia A) cPent | 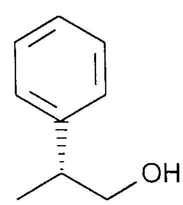 |
| 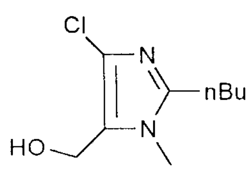 | (dia B) cPent | 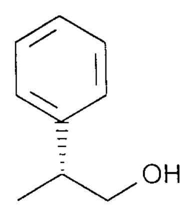 |

| A | $R^1$ | $R^2$ |
|---|---|---|
| 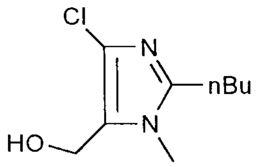 | (dia A) cHept | 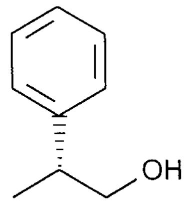 |
| 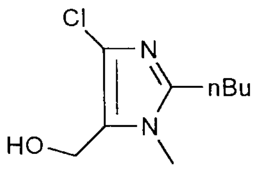 | (dia B) cHept | 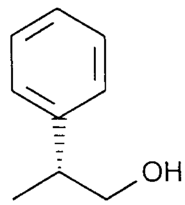 |
| 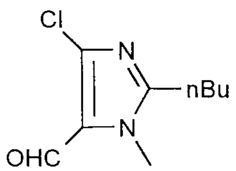 | (R&S) cPent | 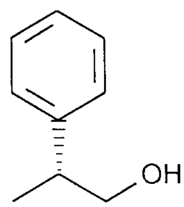 |
| 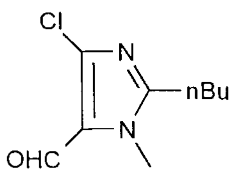 | (R&S) cHept | 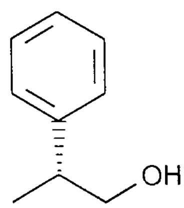 |
| 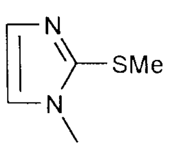 | (R&S) cPent | 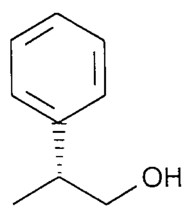 |
| 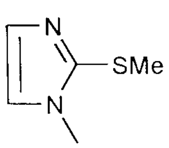 | (R&S) cPent | $CH_2$-$C_6H_5$ |
| 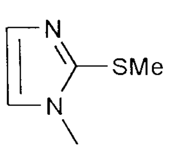 | (R&S) cHept | 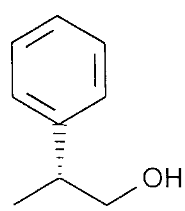 |

| A | $R^1$ | $R^2$ |
|---|---|---|
| 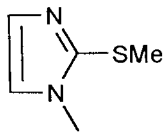 | (R&S) cHept | $CH_2$-$C_6H_5$ |
| 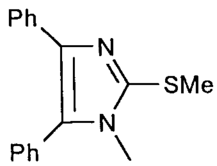 | (R&S) cPent | 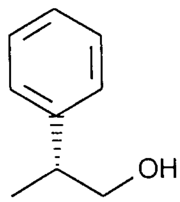 |
| 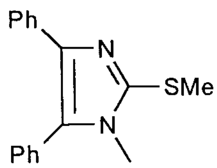 | (R&S) cPent | $CH_2$-$C_6H_5$ |
| 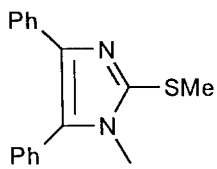 | (R&S) cHept | 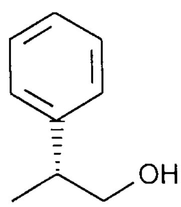 |
| 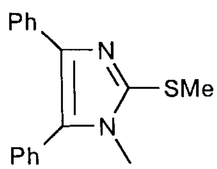 | (R&S) cHept | $CH_2$-$C_6H_5$ |
| 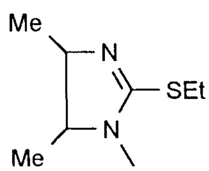 | (R&S) cPent | 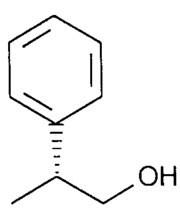 |
| 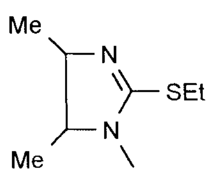 | (R&S) cPent | $CH_2$-$C_6H_5$ |

| A | $R^1$ | $R^2$ |
|---|---|---|
| 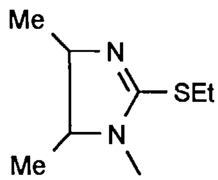 | (R&S) cHept | 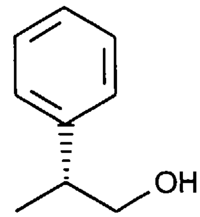 |
| 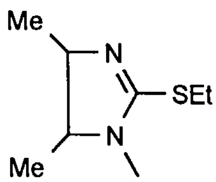 | (R&S) cHept | $CH_2$-$C_6H_5$ |
| 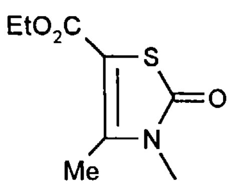 | (R&S) cPent | 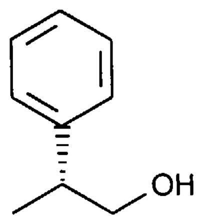 |
| 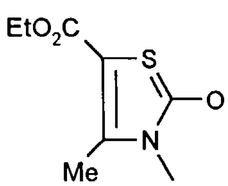 | (R&S) cPent | $CH_2$-$C_6H_5$ |
| 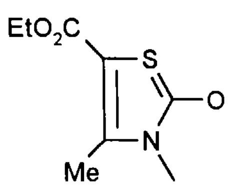 | (R&S) cHept | 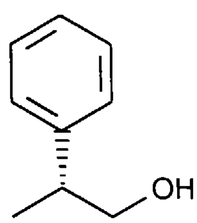 |

| A | $R^1$ | $R^2$ |
|---|---|---|
| 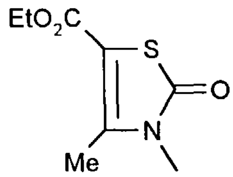 | (R&S) cHept | $CH_2$-$C_6H_5$ |
| 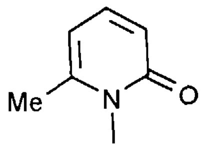 | (R&S) cPent | 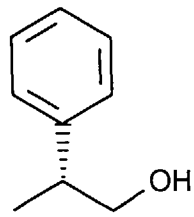 |
| 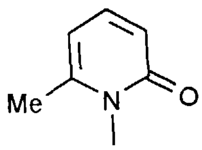 | (R&S) cPent | $CH_2$-$C_6H_5$ |
| 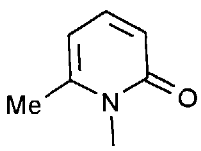 | (R&S) cHept | 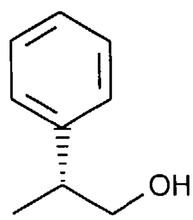 |
| 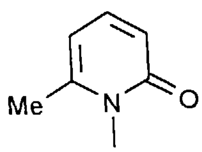 | (R&S) cHept | $CH_2$-$C_6H_5$ |
| 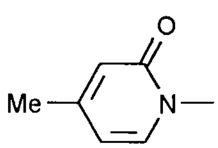 | (R&S) cPent | 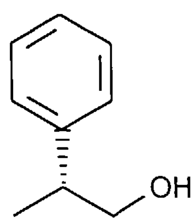 |

| A | $R^1$ | $R^2$ |
|---|---|---|
| 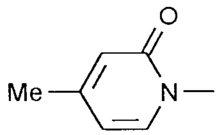 | (R&S) cPent | $CH_2$-$C_6H_5$ |
| 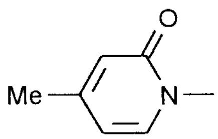 | (R&S) cHept | 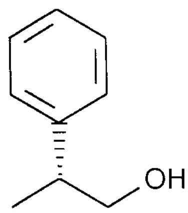 |
| 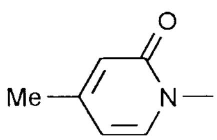 | (R&S) cHept | $CH_2$-$C_6H_5$ |
| 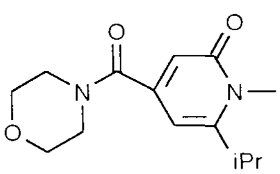 | (R&S) cPent | 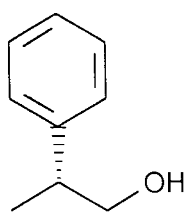 |
| 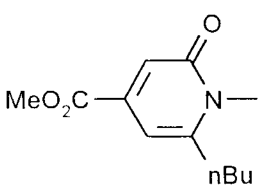 | (R&S) cPent | 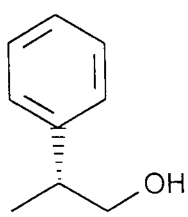 |
| 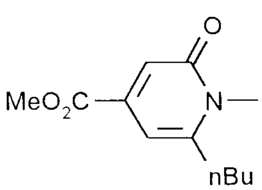 | (R&S) cPent | $CH_2$-$C_6H_5$ |

| A | $R^1$ | $R^2$ |
|---|---|---|
| 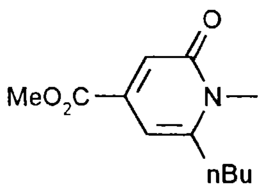 | (R&S) cHept | 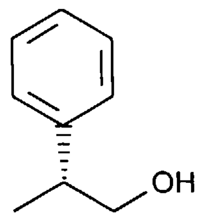 |
| 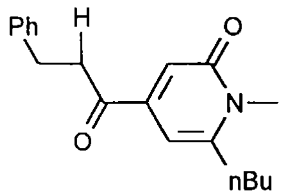 | (R&S) cPent | $CH_2$-$C_6H_5$ |
| 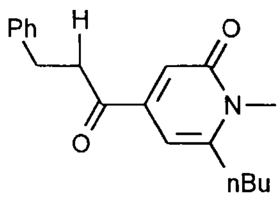 | (R&S) cHept | $CH_2$-$C_6H_5$ |
| 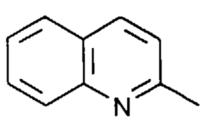 | (dia A) cPent | |
| 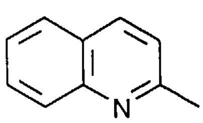 | (dia B) cPent | 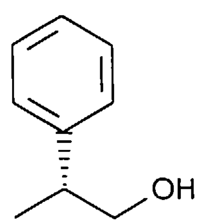 |

Außerdem wurde ein Verfahren zur Herstellung der erfindungsgemäßen Verbindungen der allgemeinen Formel (I) gefunden, dadurch gekennzeichnet, daß man
Carbonsäuren der allgemeinen Formel (II)

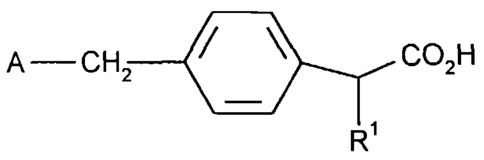

(II)

in welcher
A und $R^1$ die oben angegebene Bedeutung haben,
mit Aminen der allgemeinen Formel (III)

$$H_2N\text{-}R^2 \qquad \text{(III)}$$

in welcher
$R^2$ die oben angegebene Bedeutung hat,
in inerten Lösemitteln und in Anwesenheit von Basen und/oder Hilfsmitteln umsetzt.

Das erfindungsgemäße Verfahren kann durch folgendes Formelschema beispielhaft erläutert werden:

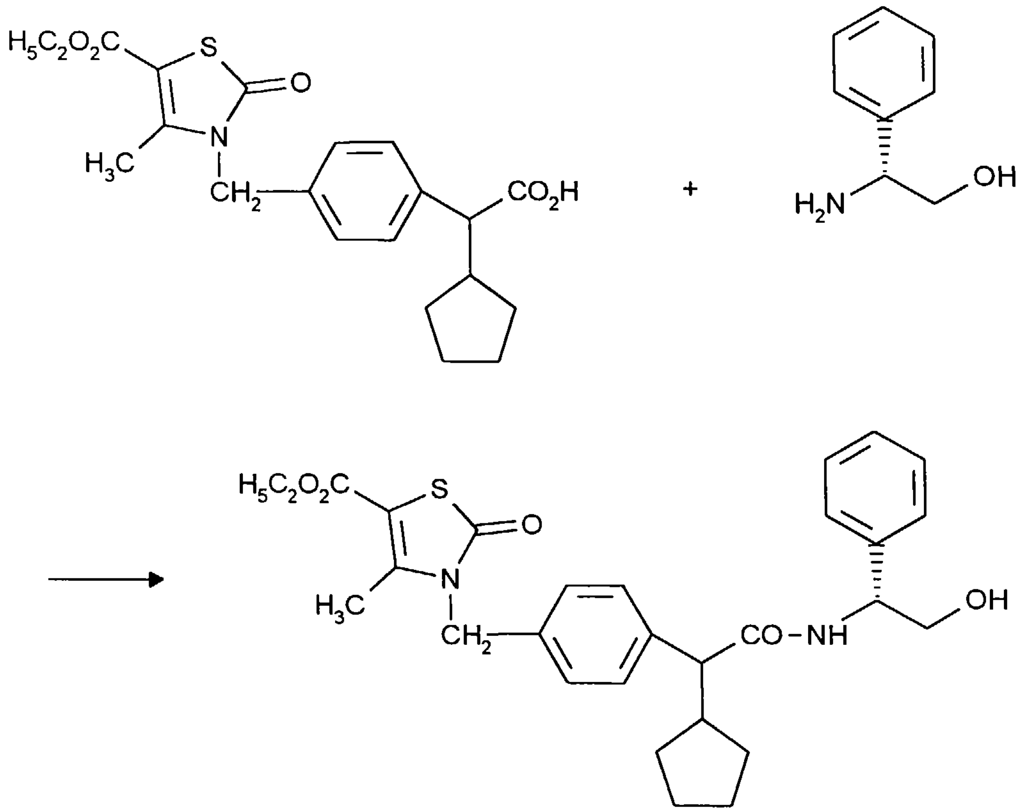

Als Lösemittel für die Amidierung eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat,

Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]-octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium und deren Hydride wie Natriumhydrid, einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II), eingesetzt.

Die Reaktion wird im allgemeinen in einem Temperaturbereich von 0°C bis 150°C, bevorzugt von +20°C bis +110°C, durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Die Amidierung kann gegebenenfalls auch über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die oben aufgeführten Basen können gegebenenfalls auch als säurebindende Hilfsmittel für die Amidierung eingesetzt werden.

Als Hilfsmittel eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Iso-butylchloroformat oder Benzotriazolyloxy-tris-(dimethylamino)phosphoniumhexa-fluorophosphat oder Phosphorsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Variation von funktionellen Gruppen wie beispielsweise Hydrolyse, Veresterung und Reduktion, sowie die Isomerentrennung und Salzbildung erfolgt nach üblichen Methoden.

Die Carbonsäuren der allgemeinen Formel (II) sind größtenteils neu und können hergestellt werden, indem man Verbindungen der allgemeinen Formel (IV)

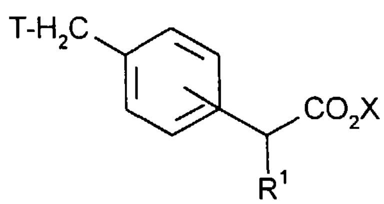

(IV),

in welcher

$R^1$ die angegebene Bedeutung hat,

T für eine typische Abgangsgruppe wie beispielsweise Chlor, Brom, Jod, Tosylat oder Mesylat, vorzugsweise für Brom, steht,
und

X für ($C_1$-$C_4$)-Alkyl steht,
mit Verbindungen der allgemeinen Formel (V)

A-H (V),

in welcher

A die angegebene Bedeutung hat,

in inerten Lösemitteln, gegebenenfalls in Anwesenheit einer Base, umsetzt und anschließend die Ester nach üblichen Methoden verseift.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether,

oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]-octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (IV), ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (III) sind an sich bekannt.

Die Verbindungen der allgemeinen Formel (IV) sind bekannt oder können in Analogie zu bekannten Methoden hergestellt werden.

Die Verbindungen der allgemeinen Formel (V) sind bekannt oder können in Analogie zu bekannten Methoden hergestellt werden.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche

befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Loch-platten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Triglyceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs ($\Delta$TG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in $\Delta$% von der ölbelasteten Kontrolle angegeben.

$$\Delta\%\ \text{Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht Triton WR-1339 (2,5 mg/kg), gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von heterocyclisch-substituierten Phenylglycinolamiden der allgemeinen Formel (I) mit einem Glucosidase- und/oder Amylasehemmer zur Behandlung von familiärer Hyperlipidaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Benutzte Abkürzungen:**

Ph = Phenyl
Me = Methyl
Et = Ethyl
cHex = Cyclohexyl
Bn = $CH_2$-$C_6H_5$
Gly =

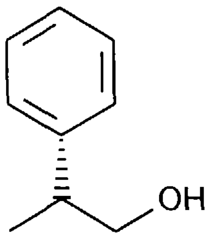

C = Cyclohexan
EE = Essigester
P = Petrolether

<u>Ausgangsverbindungen</u>

**Beispiel I**

2-Cyclopentyl-2-[4-(2-phenyl-imidazol-1-yl-methyl)phenyl]essigsäure-tert.butylester

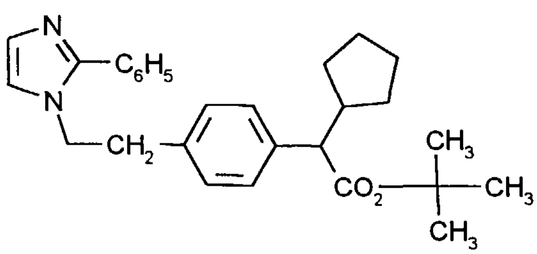

1,44 g (10 mmol) 2-Phenylimidazol werden in 10 ml DMF gelöst, mit 330 mg (11 mmol) NaH (80%ig) deprotoniert (50°C) und anschließend mit 3,5 g (10 mmol) 4-Brommethyl-2-cyclopentyl-essigsäure-tert.butylester (DE 42 00 954 A1) versetzt und über Nacht bei RT gerührt. Es wird eingeengt, in $CH_2Cl_2$ gelöst, mit $H_2O$ gewaschen, eingeengt und der Rückstand an Kieselgel chromatographiert (Cyclohexan / EE = 8:2).

Man erhält 3,2 g (75%) als farbloses Harz.

**Beispiel II**

2-Cyclopentyl-2-[4-(2-phenyl-imidazol-1-ylmethyl)phenyl]essigsäure

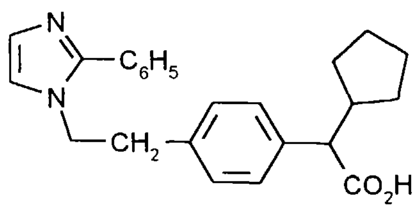

3,0 g (7 mmol) der Verbindung aus Beispiel I werden in 14 ml Dioxan gelöst, mit 2 ml konz. HCl versetzt und 5 h refluxiert. Es wird eingeengt, in $CH_2Cl_2$ aufgenommen und mit Wasser gewaschen. Man erhält 2,6 g (96%) der Titelverbindung als Öl.

In Analogie zur Vorschrift des Beispiels II werden die in der Tabelle I aufgeführten Beispiele hergestellt.

**Tabelle I:**

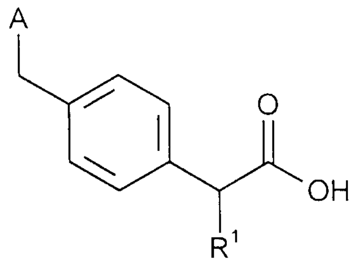

| Bsp.-Nr. | A | $R^1$ | Fp. (°C) | $R_f$ |
|---|---|---|---|---|
| III | 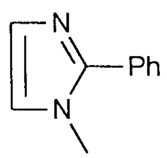 | (R&S) cPent | 224 | |
| IV | 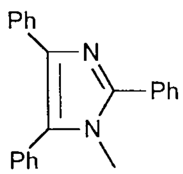 | (R&S) cPent | 194 | |
| V | 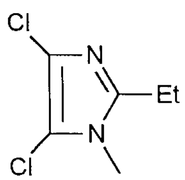 | (R&S) cPent | | |
| VI | 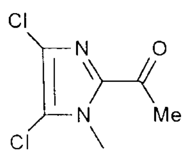 | (R&S) cPent | | 0,48<br>C/EE 1:1 |

| Bsp.-Nr. | A | $R^1$ | Fp. (°C) | $R_f$ |
|---|---|---|---|---|
| VII | 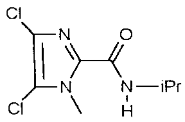 | (R&S) cPent | | |
| VIII | 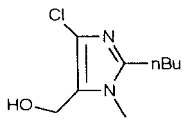 | (dia A) cPent | | |
| IX | 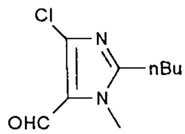 | (R&S) cPent | | |
| X | 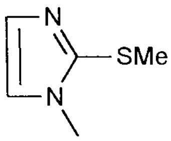 | (R&S) cPent | 172 | |
| XI | 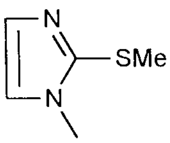 | (R&S) cHept | 177 | |
| XII | 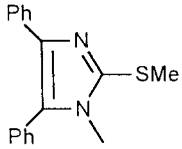 | (R&S) cPent | | 0,2<br>C/EE 1:1 |
| XIII | 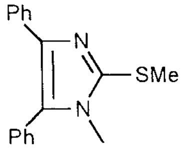 | (R&S) cHept | 206 | |

| Bsp.-Nr. | A | $R^1$ | Fp. (°C) | $R_f$ |
|---|---|---|---|---|
| XIV | 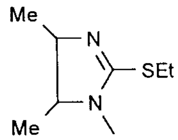 | (R&S) cPent | | 0,17 $CH_2Cl_2$/MeOH H 9:1 |
| XV | 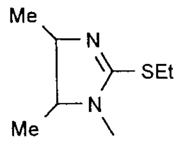 | (R&S) cHept | | 0,25 $CH_2Cl_2$/MeOH 9:1 |
| XVI | 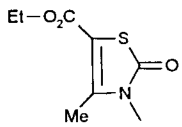 | (R&S) cPent | | 0,3 C/EE 1:1 |
| XVII | 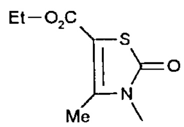 | (R&S) cHept | | 0,32 C/EE 1:1 |
| XVIII | 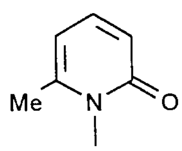 | (R&S) cPent | 203 | |
| XIX | 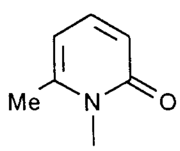 | (R&S) cHept | 192 | |
| XX | 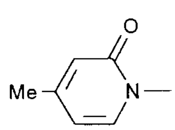 | (R&S) cPent | 156 | |

| Bsp.-Nr. | A | $R^1$ | Fp. (°C) | $R_f$ |
|---|---|---|---|---|
| XXI | 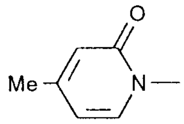 | (R&S) cHept | 200 | |
| XXII | 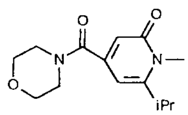 | (R&S) cPent | | 0,45 $CH_2Cl_2$/MeOH 9:1 |
| XXIII | 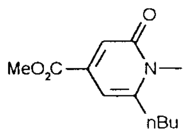 | (R&S) cPent | | |
| XXIV | 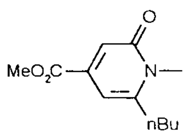 | (R&S) cHept | | |
| XXV | 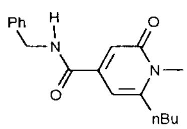 | (R&S) cPent | | |
| XXVI | 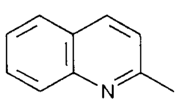 | (dia A) cPent | | |
| XXVII | 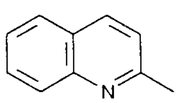 | (dia B) cPent | | |

## Herstellungsbeispiele

### Beispiel 1

2-Cyclopentyl-N-(2-hydroxy-1-(R)-phenylethyl)-2-[4-(2-phenylimidazol-1-yl-methyl)phenyl]essigsäureamid

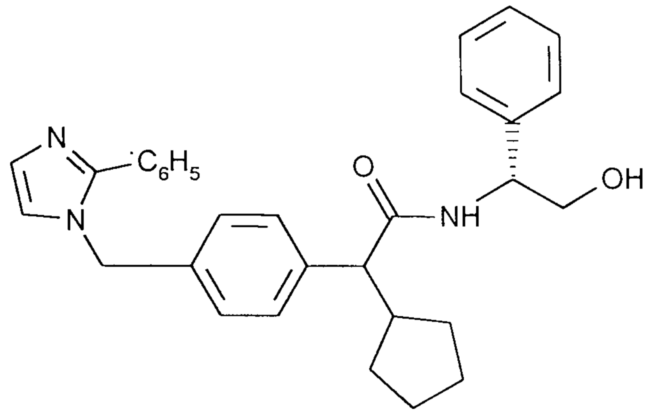

1,08 g (3 mmol) der Verbindung aus Beispiel II werden mit 0,412 g (3 mmol) R-(-)-2-Phenylglycinol (Aldrich) in 30 ml $CH_2Cl_2$ gelöst, anschließend werden 0,446 g (3,3 mmol) 1-Hydroxy-1H-benzotriazol Hydrat (Aldrich) zugegeben. Nach Zugabe von 662 mg (3,45 mmol) N'-(3-Dimethylaminopropyl)-N-ethyl-carbodiimid-hydrochlorid (Aldrich) und 0,8 ml Triethylamin wird über Nacht bei RT gerührt. Es wird mit $CH_2Cl_2$ verdünnt, je einmal mit $NH_4Cl$-Lösung und $NaHCO_3$-Lösung gewaschen, getrocknet und einrotiert. Es wird mit Cyclohexan / Essigester (1:1) chromatographiert.

Ausbeute: 1,46 g (98%).
$R_f$ = 0,17 (Cyclohexan / Essigester = 1:1)

In Analogie zur Vorschrift des Beispiels 1 werden die in der Tabelle 1 aufgeführten Verbindungen über die entsprechenden Vorstufen (analog der Beispiele I und II) hergestellt:

**Tabelle 1:**

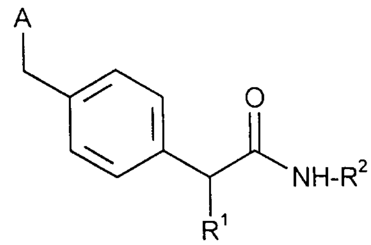

| Bsp.-Nr. | A | $R^1$ | $R^2$ | Fp. (°C) | $R_f$ |
|---|---|---|---|---|---|
| 2 | 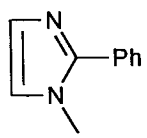 | (R&S) cPent | N-Bn | | 0,12 C/EE 4:6 |
| 3 | 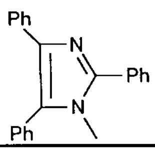 | (R&S) cPent | R-Gly | 175 | |
| 4 | 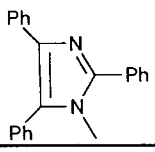 | (R&S) cPent | Bn | | 0,25 C/EE 7:3 |
| 5 | 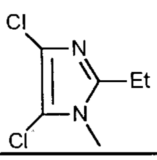 | (R&S) cPent | R-Gly | | 0,5 $CH_2Cl_2/CH_3OH$ 10:1 |
| 6 | 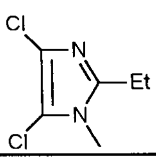 | (dia A) cPent | R-Gly | | 0,5 $CH_2Cl_2/CH_3OH$ 10:1 |
| 7 | 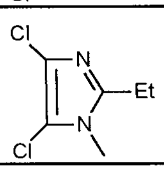 | (dia B) cPent | R-Gly | | 0,5 $CH_2Cl_2/CH_3OH$ 10:1 |
| 8 | 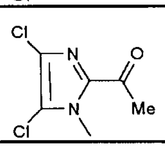 | (R&S) cPent | R-Gly | 154 | |
| 9 | 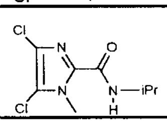 | (R&S) cPent | R-Gly | | 0,39 P / EE 1:1 |

| Bsp.-Nr. | A | $R^1$ | $R^2$ | Fp. (°C) | $R_f$ |
|---|---|---|---|---|---|
| 10 | 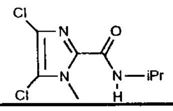 | (dia A) cPent | R-Gly | | 0,43 P/EE 1:1 |
| 11 | 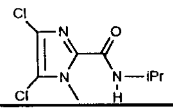 | (dia B) cPent | R-Gly | | 0,35 P/EE 1:1 |
| 12 | 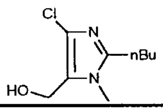 | (dia A) cPent | R-Gly | | 0,16 P/EE 4:6 |
| 13 | 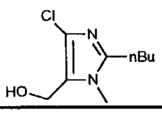 | (dia B) cPent | R-Gly | | 0,09 P/EE 4:6 |
| 14 | 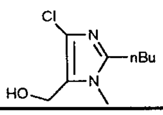 | (dia A) cHept | R-Gly | | 0,24 P/EE 4:6 |
| 15 | 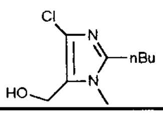 | (dia B) cHept | R-Gly | | 0,12 P/EE 4:6 |
| 16 | 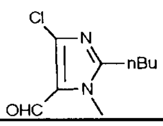 | (R&S) cPent | R-Gly | | 0,35 P/EE 1:1 |
| 17 | 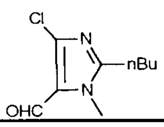 | (R&S) cHept | R-Gly | | 0,44 P/EE 1:1 |
| 18 | 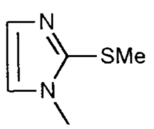 | (R&S) cPent | R-Gly | | 0,58 C/EE 4:6 |
| 19 | 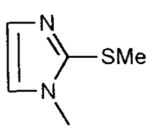 | (R&S) cPent | Bn | 105-106 | |
| 20 | 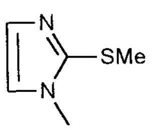 | (R&S) cHept | R-Gly | | 0,08 C/EE 4:6 |
| 21 | 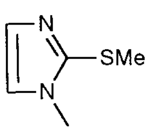 | (R&S) cHept | Bn | | 0,2 C/EE 4:6 |

| Bsp.-Nr. | A | $R^1$ | $R^2$ | Fp. (°C) | $R_f$ |
|---|---|---|---|---|---|
| 22 | 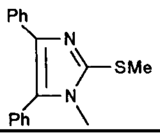 | (R&S) cPent | R-Gly | | 0,28 C/EE 1:1 |
| 23 | 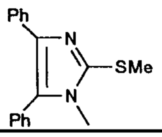 | (R&S) cPent | Bn | 119-120 | |
| 24 | 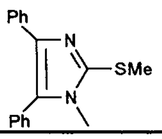 | (R&S) cHept | R-Gly | | 0,32 C/EE 1:1 |
| 25 | 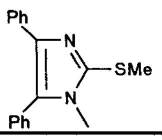 | (R&S) cHept | Bn | 168-169 | |
| 26 | 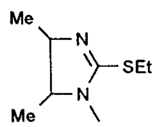 | (R&S) cPent | R-Gly | | 0,11 $CH_2Cl_2/CH_3OH$ 9:1 |
| 27 | 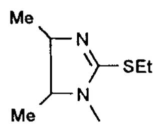 | (R&S) cPent | Bn | | 0,25 $CH_2Cl_2/CH_3OH$ 9:1 |
| 28 | 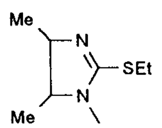 | (R&S) cHept | R-Gly | | 0,23 $CH_2Cl_2/CH_3OH$ 9:1 |
| 29 | 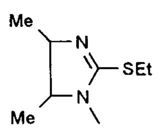 | (R&S) cHept | Bn | | 0,19 $CH_2Cl_2/CH_3OH$ 9:1 |
| 30 | 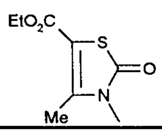 | (R&S) cPent | R-Gly | | 0,18 C/EE 1:1 |
| 31 | 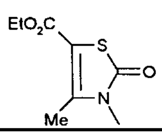 | (R&S) cPent | Bn | | 0,15 C/EE 7:3 |
| 32 | 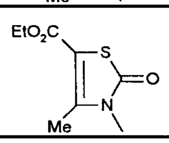 | (R&S) cHept | R-Gly | | 0,21 C/EE 1:1 |

| Bsp.-Nr. | A | $R^1$ | $R^2$ | Fp. (°C) | $R_f$ |
|---|---|---|---|---|---|
| 33 | 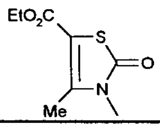 | (R&S) cHept | Bn | | 0,22 C/EE 7:3 |
| 34 | 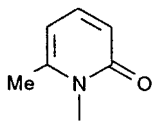 | (R&S) cPent | R-Gly | | 0,27 $CH_2Cl_2/CH_3OH$ 95:5 |
| 35 | 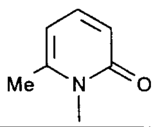 | (R&S) cPent | Bn | 173-174 | |
| 36 | 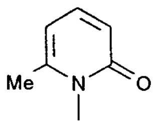 | (R&S) cHept | R-Gly | | 0,25 $CH_2Cl_2/CH_3OH$ 95:5 |
| 37 | 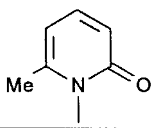 | (R&S) cHept | Bn | 175-176 | |
| 38 | 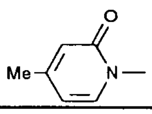 | (R&S) cPent | R-Gly | | 0,28 $CH_2Cl_2/CH_3OH$ 95:5 |
| 39 | 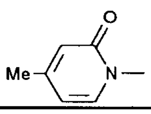 | (R&S) cPent | Bn | 146 | |
| 40 | 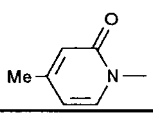 | (R&S) cHept | R-Gly | | 0,03 $CH_2Cl_2/CH_3OH$ 95:5 |
| 41 | 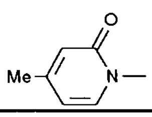 | (R&S) cHept | Bn | 186 | |
| 42 | 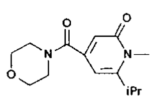 | (R&S) cPent | R-Gly | | 0,31 $CH_2Cl_2/CH_3OH$ 95:5 |
| 43 | 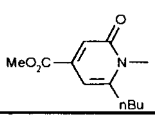 | (R&S) cPent | R-Gly | | 0,3 $CH_2Cl_2/CH_3OH$ 95:5 |
| 44 | 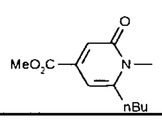 | (R&S) cPent | Bn | | 0,4 C/EE 4:6 |

| Bsp.-Nr. | A | $R^1$ | $R^2$ | Fp. (°C) | $R_f$ |
|---|---|---|---|---|---|
| 45 | 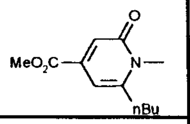 | (R&S) cHept | R-Gly | | 0,2 $CH_2Cl_2/CH_3OH$ 95:5 |
| 46 | 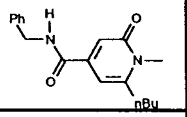 | (R&S) cPent | Bn | | 0,21 C/EE 4:6 |
| 47 | 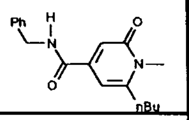 | (R&S) cHept | Bn | 172 | |
| 48 | 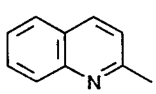 | (dia A) cPent | R-Gly | | 0,37 $CH_2Cl_2/CH_3OH$ 100:10 |
| 49 | 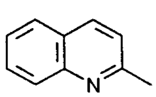 | (dia B) cPent | R-Gly | | 0,37 $CH_2Cl_2/CH_3OH$ 100:10 |

## Patentansprüche

1. Heterocyclisch-substituierte Phenylglycinolamide der allgemeinen Formel (I)

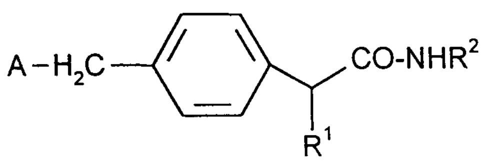

(I)

in welcher

A für Chinolyl oder für einen Rest der Formel

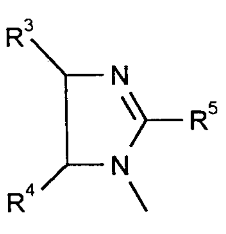

,

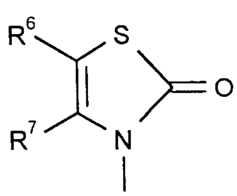

oder

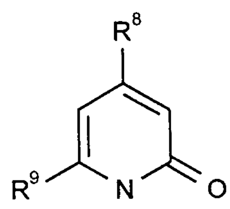

steht, in welcher

$R^3$, $R^4$, $R^6$ und $R^7$ gleich oder verschieden sind und Wasserstoff, Phenyl, Halogen, Formyl, Carboxyl, geradkettiges oder verzweigtes Alkoxycarbonyl mit bis zu 4 Kohlenstoffatomen oder geradkettiges oder verzweigtes Alkyl mit bis zu 4 Kohlenstoffatomen bedeuten, das gegebenenfalls durch

Hydroxyl substituiert ist,

$R^5$ Phenyl, geradkettiges oder verzweigtes Alkyl, Acyl oder Alkylthio mit jeweils bis zu 6 Kohlenstoffatomen oder eine Gruppe der Formel $-CO-NR^{10}R^{11}$ bedeutet,
worin

$R^{10}$ und $R^{11}$ gleich oder verschieden sind und Wasserstoff oder geradkettiges oder verzweigtes Alkyl mit bis zu 5 Kohlenstoffatomen bedeuten,

$R^8$ und $R^9$ gleich oder verschieden sind und Wasserstoff, geradkettiges oder verzweigtes Alkyl oder Alkoxycarbonyl mit jeweils bis zu 6 Kohlenstoffatomen oder einen Rest der Formel $-CO-R^{12}$ bedeuten,
worin

$R^{12}$ Morpholinyl oder den Rest der Formel
$—NH—CH_2—C_6H_5$ oder

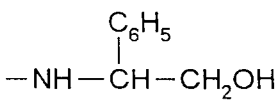

bedeutet,

$R^1$ für Cycloalkyl mit 3 bis 8 Kohlenstoffatomen steht, oder für geradketiges oder verzweigtes Alkyl mit bis zu 10 Kohlenstoffatomen steht,

$R^2$ für einen Rest der Formel

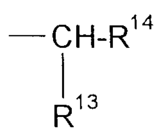

steht,
worin

$R^{13}$ Wasserstoff oder einen Rest der Formel $CH_2$-OH bedeutet,

$R^{14}$ Phenyl bedeutet, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

**2.** Heterocyclisch-substituierte Phenylglycinolamide der Formel nach Anspruch 1
in welcher

A für Chinolyl oder
für einen Rest der Formel

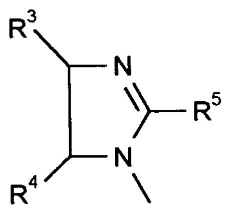,
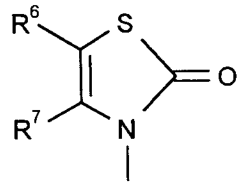 oder 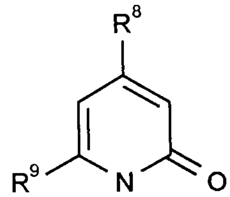

steht,
in welcher

$R^3$, $R^4$, $R^6$ und $R^7$ gleich oder verschieden sind und Wasserstoff, Phenyl, Fluor, Chlor, Brom, Formyl, geradkettiges oder verzweigtes Alkoxycarbonyl mit bis zu 3 Kohlenstoffatomen oder geradkettiges oder verzweigtes Alkyl mit bis zu 3 Kohlenstoffatomen bedeuten, das gegebenenfalls durch Hydroxyl substituiert ist,

$R^5$ Phenyl, geradkettiges oder verzweigtes Alkyl, Acyl oder Alkylthio mit jeweils bis zu 5 Kohlenstoffatomen oder eine Gruppe der Formel $-CO-NR^{10}R^{11}$ bedeutet,
worin

$R^{10}$ und $R^{11}$ gleich oder verschieden sind und Wasserstoff oder geradkettiges oder verzweigtes Alkyl mit bis zu 5 Kohlenstoffatomen bedeuten,

$R^8$ und $R^9$ gleich oder verschieden sind und Wasserstoff, geradkettiges oder verzweigtes Alkyl oder Alkoxycarbonyl mit jeweils bis zu 5 Kohlenstoffatomen oder einen Rest der Formel $-CO-R^{12}$ bedeuten,
worin

$R^{12}$ Morpholinyl oder den Rest der Formel
$-NH-CH_2-C_6H_5$ oder

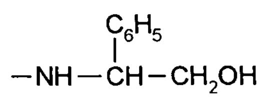

bedeutet,

$R^1$ für Cyclopropyl, Cyclobutyl, Cyclopentyl, Cyclohexyl oder Cycloheptyl steht, oder
für geradkettiges oder verzweigtes Alkyl mit bis zu 8 Kohlenstoffatomen steht,

$R^3$ für einen Rest der Formel

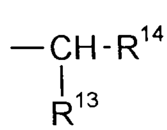

steht,
worin

$R^{13}$ Wasserstoff oder einen Rest der Formel $CH_2$-OH bedeutet,

$R^{14}$ Phenyl bedeutet, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen

substituiert ist,

und deren Salze.

**3.** Heterocyclisch-substituierte Phenylglycinolamide der Formel nach Anspruch 1 in welcher

A für Chinolyl oder für einen Rest der Formel

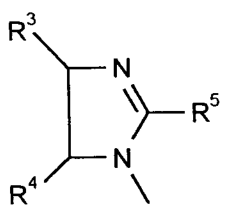
,
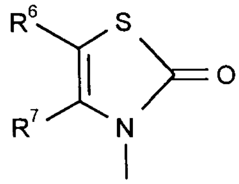
oder
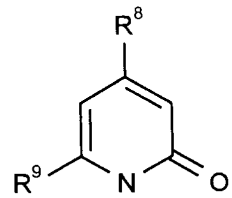

steht, in welcher

$R^3$, $R^4$, $R^6$ und $R^7$ gleich oder verschieden sind und Wasserstoff, Phenyl, Chlor, Formyl, Methoxycarbonyl, Ethoxycarbonyl oder Methyl oder Ethyl bedeuten, das gegebenenfalls durch Hydroxyl substituiert ist,

$R^5$ Phenyl, Methylthio, Acetyl, Ethylthio oder geradkettiges oder verzweigtes Alkyl mit bis zu 4 Kohlenstoffatomen oder eine Gruppe der Formel $-CO-NR^{10}R^{11}$ bedeutet, worin

$R^{10}$ und $R^{11}$ gleich oder verschieden sind und Wasserstoff oder geradkettiges oder verzweigtes Alkyl mit bis zu 3 Kohlenstoffatomen bedeuten,

$R^8$ Methyl, Methoxycarbonyl, Ethoxycarbonyl oder den Rest der Formel

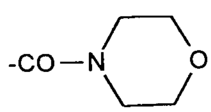
,

$-CO-NH-CH_2-C_6H_5$ oder

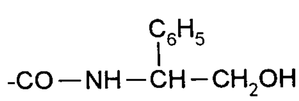

bedeutet, und

$R^9$ Wasserstoff, Methyl, Propyl oder Butyl bedeutet,

und deren Salze.

**4.** Heterocyclisch-substituierte Phenylglycinolamide nach Anspruch 1 bis 3 als Arzneimittel.

**5.** Verfahren zur Herstellung von heterocyclisch-substituierten Phenylglycinolamiden nach Anspruch 1 bis 3, dadurch gekennzeichnet, daß man

Carbonsäuren der allgemeinen Formel (II)

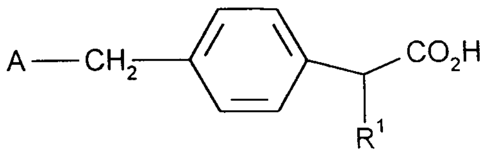

(II)

in welcher
A und $R^1$ die oben angegebene Bedeutung haben,
mit Aminen der allgemeinen Formel (III)

$$H_2N\text{-}R^2 \qquad (III)$$

in welcher
$R^2$ die oben angegebene Bedeutung hat,
in inerten Lösemitteln und in Anwesenheit von Basen und/oder Hilfsmitteln umsetzt.

**6.** Arzneimittel enthaltend mindestens ein heterocyclisch-substituiertes Phenylglycinolamid nach Anspruch 1 bis 3 und ein pharmakologisch unbedenkliches Formulierungshilfsmittel.

**7.** Arzneimittel nach Anspruch 6 zur Behandlung von Atherosklerose.

**8.** Verwendung von heterocyclisch-substiuierten Phenylglycinolamiden nach Anspruch 1 bis 3 zur Herstellung von Arzneimitteln.

**9.** Verwendung nach Anspruch 8, zur Herstellung von antiatherosklerotischen Arzneimitteln.

**10.** Verwendung von heterocyclisch-substituierten Phenylglycinolamiden nach Anspruch 1 bis 3, zur Verminderung oder vollständigen Inhibierung oder Bildung und/oder der Freisetzung von ApoB-100-assoziierten Lipoproteinen.

Europäisches Patentamt

# EUROPÄISCHER RECHERCHENBERICHT

Nummer der Anmeldung
EP 97 10 5596

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X,D | EP 0 513 533 A (BAYER AG) 19.November 1992 * siehe Überlappung der Formeln sowie insbesondere Seite 93, Tab.XXI, Seite 100, Tab.XXVI und Seite 102, Tab.XXVIII * * das ganze Dokument * --- | 1-10 | C07D233/58 A61K31/415 C07D233/68 C07D233/84 C07D277/34 C07D213/64 C07D215/14 A61K31/435 A61K31/47 |
| X,D | EP 0 560 162 A (BAYER AG) 15.September 1993 * siehe insbesondere Seite 24, Tabelle 4, Beispiele 12 und 13 * * das ganze Dokument * --- | 1-10 | |
| X,D | EP 0 560 163 A (BAYER AG) 15.September 1993 * siehe Beispiele 1-11, 14-16 * * das ganze Dokument * --- | 1-10 | |
| X | EP 0 622 358 A (BAYER AG) 2.November 1994 * siehe Beispiele 1 - 19 * * das ganze Dokument * --- | 1-10 | RECHERCHIERTE SACHGEBIETE (Int.Cl.6) |
| Y | EP 0 565 986 A (BAYER AG) 20.Oktober 1993 * das ganze Dokument * --- | 1-10 | C07D |
| Y | EP 0 705 831 A (BAYER AG) 10.April 1996 * das ganze Dokument * --- | 1-10 | |
| Y | EP 0 542 059 A (BAYER AG) 19.Mai 1993 * das ganze Dokument * --- | 1-10 | |
| Y | EP 0 624 583 A (BAYER AG) 17.November 1994 * das ganze Dokument * --- | 1-10 | |
| Y | EP 0 630 896 A (BAYER AG) 28.Dezember 1994 * das ganze Dokument * --- | 1-10 | |
| Y | EP 0 610 698 A (BAYER AG) 17.August 1994 * das ganze Dokument * --- | 1-10 | |
| | -/-- | | |

Der vorliegende Recherchenbericht wurde für alle Patentansprüche erstellt

| Recherchenort | Abschlußdatum der Recherche | Prüfer |
|---|---|---|
| MÜNCHEN | 24.Juli 1997 | Stellmach, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 97 10 5596

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP 0 608 709 A (BAYER AG) 3.August 1994<br>* das ganze Dokument *<br>--- | 1-10 | |
| P,X | EP 0 716 082 A (BAYER AG) 12.Juni 1996<br>* das ganze Dokument *<br>----- | 1-10 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| MÜNCHEN | 24.Juli 1997 | Stellmach, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)